(19) Europäisches Patentamt
European Patent Office
Office européen des brevets

(11) Publication number: **0 059 009**
**A1**

(12) # EUROPEAN PATENT APPLICATION

(21) Application number: **82200132.7**

(22) Date of filing: **03.02.82**

(51) Int. Cl.³: **C 07 D 401/06**, A 61 K 31/47

(30) Priority: **23.02.81 NL 8100877**

(43) Date of publication of application: **01.09.82**
**Bulletin 82/35**

(84) Designated Contracting States: **BE CH DE FR GB IT LI NL**

(71) Applicant: **ACF Chemiefarma N.V., Straatweg 2, NL-3604 BB Maarssen (NL)**

(72) Inventor: **Trijzelaar, Hans Barend, Jan Ligthartlaan 43, NL-3706 VE Zeist (NL)**
Inventor: **de Bode, Ronus, Putterlaan 34, NL-3722 WH Bilthoven (NL)**
Inventor: **Welle, Hendricus Bernardus A., Maire Hofstedelaan 12, NL-3601 BR Maarssen (NL)**

(74) Representative: **Kupecz, Arpad et al, Octrooibureau Los en Stigter B.V. Postbox 20052, NL-1000 HB Amsterdam (NL)**

(54) **Pharmaceutical compositions containing quinoline derivatives as active component, a method for the preparation of such compositions, new quinoline derivatives and a method for the preparation thereof.**

(57) A pharmaceutical composition containing a quinoline derivative as active component in combination with a pharmaceutically suitable carrier or vehicle.

The active component is a compound having the formula

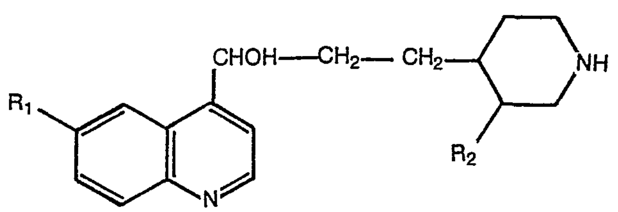

in which $R_1$ is H or a $C_{1-6}$ alkoxy or $C_{3-6}$ alkoxyalkoxy group and $R_2$ represents ethyl or vinyl, or a pharmaceutically acceptable salt thereof.

Said pharmaceutical compositions have desirable effects on the cardiovascular system, such as anti-hypertensive, antithrombotic, vasodilatory and in particular anti-arrhythmic activity.

Furthermore, the invention comprises a method for the preparation of said pharmaceutical compositions. Active compounds having the above formula and a process for the preparation thereof are also covered by the present invention.

EP 0 059 009 A1

ACTORUM AG

PHARMACEUTICAL COMPOSITIONS CONTAINING QUINOLINE DERIVATIVES AS ACTIVE COMPONENT, A METHOD FOR THE PREPARATION OF SUCH COMPOSITIONS, NEW QUINOLINE DERIVATIVES AND A METHOD FOR THE PREPARATION THEREOF.

The invention relates to pharmaceutical compositions containing quinoline derivatives as active component, a method for the preparation of such compositions, new quinoline derivatives and a method for the preparation thereof.

In French Patent 73,41043 (Publ. No. 2,206,944) quinoline derivatives of formula 2 are described,

2 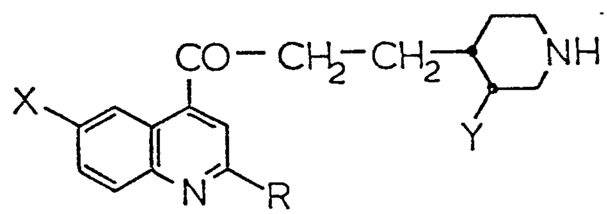

in which X is hydrogen or methoxy, Y is hydrogen, ethyl or vinyl and R is $C_{1-4}$ alkyl, cycloalkyl or optionally substituted aralkyl or aryl, which compounds may be used for the treatment and prophylaxis of cardiovascular affections.

From Ann. Pharm. Fr. 24 39 (1966) the pharmacodynamic properties of quinicine (formula 3),

3 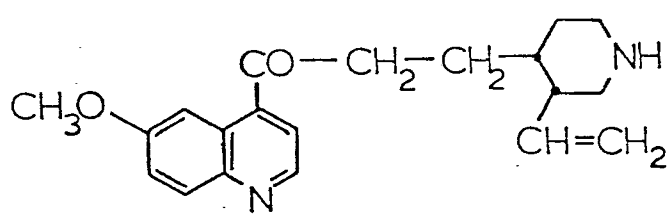

also named viquidil, are known, in particular in the field of CNS, and the hypotensive, vasodilative and anti-spasmodic activities.

In British Patent 1,294,538 the use of viquidil in the treatment of cerebral vessel injury, cerebrovascular insufficiency and memory deficiency in humans is described.

In Dutch Patent Application 77,06614 quinoline derivatives are described with formula 4,

4 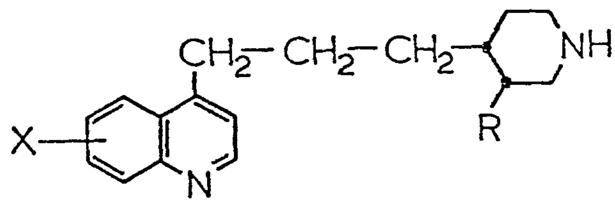

in which R is hydrogen, $C_{1-4}$ alkyl or $C_{2-4}$ alkenyl and X is hydrogen, halogen, $C_{1-4}$ alkyl, alkoxy, or alkylthio, trifluoromethyl, nitro, hydroxy, an amino group optionally substituted by one or two $C_{1-4}$ alkyl groups, or $C_{1-4}$ acyl or alkylsulfonyl group, which compounds have a serotonin uptake inhibiting effect and anti-arrhythmic activity.

The German Patent Application DT 2,949,993 discloses the compounds of formula 5,

5 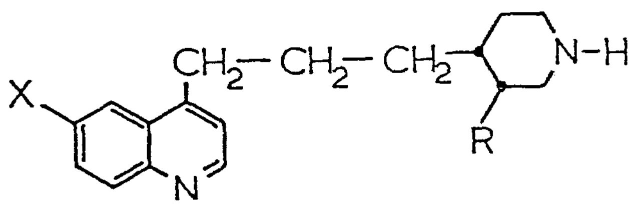

wherein R and X are both H; or R is vinyl or ethyl; and X is H or methoxy. Said compounds are used for treatment of anxiety.

The U.S. Patents 3,873,549 and 3,914,235 disclose compounds of formula 6,

6 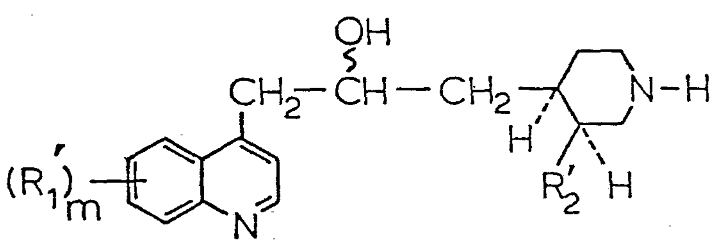

and their antipodes and racemates, where m = 0, 1 or 2; $R_1'$ = OH, halogen, $CF_3$, methyl, ethyl, propyl, butyl or methoxy or, when m is 2, $R_1'$ with an adjacent $R_1'$ may also be

methylenedioxy; and $R_2'$ is vinyl or ethyl, as intermediates for quinine and quinidine compounds of known use as anti-malarials and anti arrhythmics, whereas such compounds showed bactericidal activity.

In J. Am. Chem. Soc. 44 1098 (1922) the preparation of compounds of formula 7 is described,

7

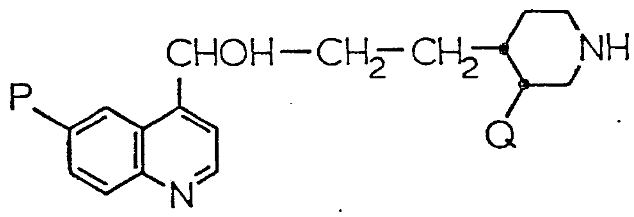

in which P is H or methoxy and O is ethyl, by simultaneous catalytic reduction of the ketone and vinyl group of cinchonicine or quinicine respectively giving rise to dihydrocinchonicinol or dihydroquinicinol.

A.D. Ainly and H. King, Proc. Roy. Soc. (London) B 125, 49 (1938); Chem. Abstr. 32, 4219b, (1938) reported that above dihydroquiniconol did not show anti-malaria activity.

In Germant Patent 330,813 (1920) the preparation of cinchonicinol (cinchotoxol) of formula 7 in which P is H and Q is vinyl is described but no biological activity is claimed.

Surprisingly, it has now been found, that quinoline derivatives of formula 1 substituted at the 4-position and optionally at the 6-position, and in which the substituent at the 4-position contains a 3-(3-substituted-4-piperidyl) propanol-1 group, possess unexpected pharmacological properties, namely desirable effects on the cardiovascular system such as anti-hypertensive, anti-thrombotic, vasodilatory and anti-arrhythmic activity. The compounds are particularly useful for the use in medicines having anti-hypertensive and anti-arrhythmic activity.

The substituents at the 3- and 4-position of the piperidine ring are in the cis position.

Accordingly the invention is concerned with a pharmaceutical composition containing a quinoline derivative as active component, characterized in that it contains as

active component at least a compound having formula 1,

1 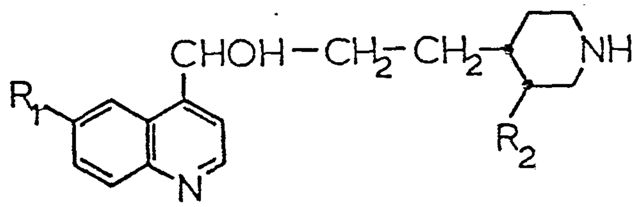

in which $R_1$ is H or a $C_{1-6}$ alkoxy or a $C_{3-6}$ alkoxyalkoxy and $R_2$ represents ethyl or vinyl, or a pharmaceutically acceptable salt thereof, in combination with a pharmaceutically suitable carrier or vehicle.

Furthermore, the invention relates to a method for the preparation of a pharmaceutical composition, characterized in that at least a compound of formula 1, in which $R_1$ and $R_2$ are as defined above, or a pharmaceutically acceptable salt thereof, is brought into a form suitable for therapeutic purposes.

The present invention also deals with new compounds of formula 1 per se, or a salt thereof, in which $R_1$ is a $C_{1-6}$ alkoxy or a $C_{3-6}$ alkoxyalkoxy and $R_2$ is vinyl and new compounds of formula 1, in which $R_1$ is a $C_{2-6}$ alkoxy or a $C_{3-6}$ alkoxyalkoxy and $R_2$ is ethyl.

Finally, the present invention relates to a method for the preparation of a compound of formula 1, in which $R_1$ and $R_2$ are defined as above, characterized in that such a compound is prepared in a manner known per se for the synthesis of analogous compounds.

So our invention relates to propanol-1-quinoline derivatives of formula 1, whereas was found that a so-called propanol-2-quinoline derivative of formula 6 as mentioned in the U.S. Patents 3,873,549 and 3,914,235, in which m = 1 and $R_1'$ is a methoxy group in the 6-position and $R_2'$ is ethyl (formula 6a)

6a 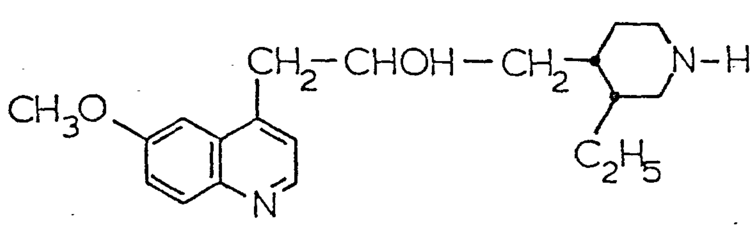

was inactive in our anti-arrhythmic screen using the electro-stimulation test described hereafter.

## Preparation

The compounds of formula 1 may be prepared using methods described in the literature per se. Compounds of formula 1, in which $R_2$ is vinyl can be prepared from cinchonicine derivatives of formula 8

8

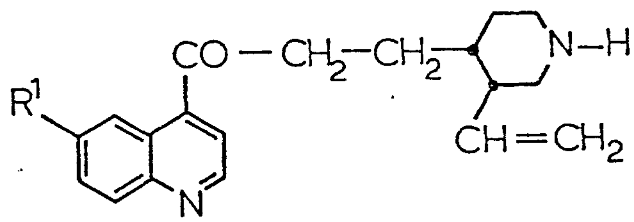

which are substituted in position 6 with $R_1$ is hydrogen or alkoxy or alkoxyalkoxy by a selective reduction using sodium borohydride, which selectively reduces the ketone group to an alcohol group whereas the vinyl group remains unattacked.

This reduction is advantageously carried out at a temperature of about -5 to -10°C in a suitable solvent, like alcohol, c.q. isopropylalcohol.

## Example I

Quinicinol-1.bifumarate (reaction 1)

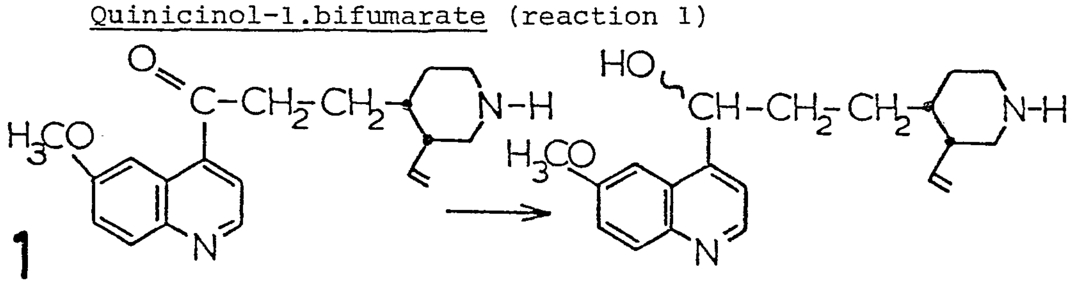

1

Quinicine, 73 g (225 mmol) was dissolved in 350 ml of isopropyl alcohol, after which the mixture was cooled to -10°C. A solution of 10.0 g (263 mmol) of sodium borohydride in 225 ml isopropyl alcohol was then added in such a way that the temperature did not exceed -5°C.

The mixture was stirred for another hour at -10°C till the conversion was substantially completed. The conversion was followed with thin layer chromatography.

The mixture was then allowed to rise to room temperature, after which 400 ml of water was added. The mixture was extracted three times with 400 ml of chloroform. The collected chloroform fractions were dried over magnesium sulphate, filtered and evaporated at reduced pressure, which afforded quinicinol-1 as an oil. The base was converted to its bifumarate (mol.ratio 1 : 1) with an equivalent amount of fumaric acid. The bifumarate had a melting point of 168-170°C. The yield was 54.0 g quinicinol-1 .bifumarate (55%).

In the same way were prepared the following compounds: (reaction 2)

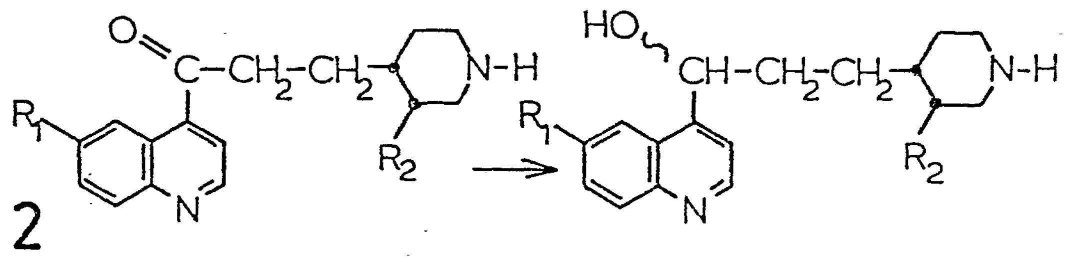

2

| Example | $R_1$ | $R_2$ | m.p°C/salt |
|---|---|---|---|
| II | $CH_3O$ | $CH_2-CH_3$ | 176-178 BF |
| III | H | $CH = CH_2$ | 138-140 BF |
| IV | H | $CH_2-CH_3$ | oil |
| V | $C_2H_5O$ | $CH_2-CH_3$ | 184-186 BF |
| VI | $n-C_3H_7O$ | $CH_2-CH_3$ | 160 TO |
| VII | $i-C_5H_{11}O$ | $CH_2-CH_3$ | 152 TO |
| VIII | $CH_3O-(CH_2)_2-O$ | $CH_2-CH_3$ | 128-130 TO |

TO = tetraoxalate (mol.ratio 1 : 2)

BF = bifumarate (mol.ratio 1:1)

## PHARMACOLOGY

**Effectiveness of the compounds of Examples in the Guinea Pigs Electrostimulation Test.**

Arrhythmias are induced in guinea pigs by electrostimulation of the right ventricle of the heart. The animals are anaesthesized with urethane (1.2 g/kg i.p.) and artificially respirated before a needle electrode is inserted in the right venticle of the heart. Substances are given intraduodinally 30 min. before the stimulation at a standard dose of 32 mg/kg.

The voltage needed for induction of extra systoles in control animals (n = 6) is compared with that required for induction of arrhythmias in treated animals (n = 6).

This method is based on the work of L. Szekeres and G.J. Papp, Naunyn-Schmiedebergs Arch. Exp. Path. Pharmak., 245, 70 (1963).

In the table the results of certain compounds of the invention are mentioned, which have been carried out according to the method escribed above.

The numbers of the compounds correspond with those of the Examples.

| Compound no. | Dose mg/kg | Percent increase in voltage (%) required for arrhythmia |
|---|---|---|
| Reference compound of formula 6a | 16 | 0 |
| I | 32 | 114 |
| | 16 | 25 |
| II | 32 | 176 |
| | 16 | 33.9 |
| III | 32 | 35 |
| VII | 23.5 | 85 |

CLAIMS

1. A pharmaceutical composition containing a quinoline derivative as active component, characterized in that it contains as active component at least a compound having formula 1,

1 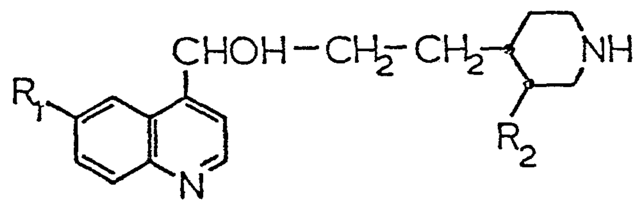

in which $R_1$ is H or a $C_{1-6}$ alkoxy or $C_{3-6}$ alkoxyalkoxy group and $R_2$ represents ethyl or vinyl, or a pharmaceutically acceptable salt thereof, in combination with a pharmaceutically suitable carrier or vehicle.

2. A method for the preparation of a pharmaceutical composition, characterized in that at least a compound of formula 1, in which $R_1$ and $R_2$ are as defined above, or a pharmaceutically acceptable salt thereof, is brought into a form suitable for therapeutic purposes.

3. A compound of formula 1 or a salt thereof, in which $R_1$ is a $C_{1-6}$ alkoxy or $C_{3-6}$ alkoxyalkoxy and $R_2$ represents vinyl.

4. A compound of formula 1 or a salt thereof, in which $R_1$ is a $C_{2-6}$ alkoxy or $C_{3-6}$ alkoxyalkoxy and $R_2$ represents ethyl.

5. A method for the preparation of a compound of formula 1, in which $R_1$ and $R_2$ are as defined in claim 1, characterized in that such a compound is prepared in a manner known per se for the synthesis of analogous compounds.

6. Compounds, pharmaceutical compositions and processes as described in the specification and in the examples.

European Patent Office

0059009
Application number

## EUROPEAN SEARCH REPORT

EP 82 20 0132

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| D,X | JOURNAL OF THE AMERICAN CHEMICAL SOCIETY, vol. 44, no. 5, May 1922 WASHINGTON (US) M. HEIDELBERGER et al.: "Syntheses in the cinchona series. X. Dihydrocinchonicinols and the dihydroquinicinols", pages 1098-1107 * entire article * --- | 1,4 | C 07 D 401/06<br>A 61 K 31/47 |
| D,X | PROCEEDINGS OF THE ROYAL SOCIETY, B125, 1938 LONDON (GB) A.D. AINLEY et al.: "Antiplasmodial action and chemical constitution. Part II. Some simple synthetic analogues of quinine and cinchonine" pages 60-92 * compounds of formula III * & CHEMICAL ABSTRACTS, vol. 32, 1938 abstract 4219b --- | 1,4 | TECHNICAL FIELDS SEARCHED (Int. Cl. 3)<br>C 07 D 401/00<br>A 61 K 31/00 |
| E | EP - A - 0 053 964 (PHARMINDUSTRIE) 16-06-1982 * entire document * --------- | 1-6 | |

The present search report has been drawn up for all claims

| Place of search | Date of completion of the search | Examiner |
|---|---|---|
| The Hague | 16-06-1982 | ALFARO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82